# NEUE EUROPÄISCHE PATENTSCHRIFT

(11) **EP 2 134 992 B2**
(45) Veröffentlichungstag und Bekanntmachung des Hinweises auf die Entscheidung über den Einspruch: **14.08.2019**
(45) Hinweis auf die Patenterteilung: 17.11.2010
(21) Anmeldenummer: 08716101.4
(22) Anmeldetag: 28.02.2008
(51) Int. Cl.: F16K 1/50

(54) **REGULIERVENTIL**
REGULATING VALVE
SOUPAPE DE REGLAGE

(30) Priorität: 16.03.2007 DE 202007004104 U
(43) Veröffentlichungstag der Anmeldung: 23.12.2009
(73) Patentinhaber: Dorma GmbH + Co. KG, 58256 Ennepetal (DE)
(72) Erfinder: WINKLER, Bernd, 58339 Breckerfeld (DE)
(74) Vertreter: Hoefer & Partner Patentanwälte mbB
(86) Internationale Anmeldenummer: PCT/EP2008/001572
(87) Internationale Veröffentlichungsnummer: WO 2008/113462

(56) Entgegenhaltungen:
- EP-A- 0 943 850
- EP-A- 1 491 801
- WO-A-2004/003324
- DE-A1- 1 575 314
- GB-A- 1 177 108
- GB-A- 1 429 299
- US-A- 4 148 111

## Beschreibung

Die Erfindung betrifft ein Regulierventil, das insbesondere in eine mit einem Innengewinde ausgeführte Bohrung eines hydraulischen Türschließers befestigbar ist, mit einem Grundkörper, der einen Regulierbereich aufweist, einer Verdrehsicherung und einem Gewinde, das in das Innengewinde der Bohrung anordbar ist.

In der DE 102 28 872 B4 ist ein Regulierventil für einen Türschließer offenbart, der aus einem Grundkörper und einem Regulierbereich besteht. Am Grundkörper befindet sich zur Regulierung des Regulierventils ein Gewinde, das in einer Bohrung des Türschließers eindrehbar ist. Der Regulierbereich ist hierbei hülsenartig ausgeführt und über eine Kugelgelenkverbindung mit dem Grundkörper verbunden. Um ein ungewolltes Verdrehen des Regulierventils, insbesondere des Regulierbereiches zu verhindern, ist eine am Regulierbereich angeordnete Verdrehsicherung vorgesehen, die als Ansatz oder Vorsprung ausgebildet ist. Dieser Ansatz ist in einer Nut innerhalb des Gehäuses des Türschließers längsbeweglich geführt. In der EP 1 491 801 A1 ist ein Regulierventil offenbart, mit einem Grundkörper, der einen Regulierbereich aufweist, einer Verdrehsicherung und einem Gewinde, das in das Innengewinde einer Bohrung anordbar ist.

US 4,148,111 A zeigt ein Regulierventil für einen hydraulischen Türschließer. Der Ventilkörper ist dabei zweiteilig aufgebaut und über ein Gewinde in das Gehäuse des Türschließers eingesetzt.

DE 1575314 A betrifft ein selbstsicherndes Befestigungselement, bestehend aus einem mit Gewinde versehenen Schaft mit einer länglichen Ausnehmung einschließlich eines darin eingelassenen Streifens aus federnd nachgiebigem Material. Des Weiteren bezieht sich die Druckschrift auf ein Verfahren zur Herstellung eines solchen Befestigungselementes.

Es ist Aufgabe der vorliegenden Erfindung, ein Regulierventil der eingangs beschriebenen Art zu schaffen, das einfach gestaltet ist, wobei ein ungewolltes Verstellen des Regulierventils während des Betriebes ausgeschlossen ist.

Zur Lösung dieser Aufgabe wird ein Regulierventil mit den Merkmalen des Patentanspruches 1 vorgeschlagen. In den abhängigen Patentansprüchen sind bevorzugte Weiterbildungen ausgeführt.

Dazu ist erfindungsgemäß vorgesehen, dass die Verdrehsicherung zumindest eine Nut aufweist, die am Gewinde angeordnet ist und ein elastisches Element aufnimmt, wobei der Grundkörper an der dem Ventilsitz zugewandten Seite einen Hohlraum aufweist, in den sich der Regulierbereich erstreckt und der Regulierbereich am Grundkörper befestigt ist. Das Regulierventil ist innerhalb des Türschließers eingeschraubt, wobei das Schließverhalten des Türschließers am Regulierventil eingestellt werden kann. Das erfindungsgemäße Regulierventil weist aufgrund der Verdrehsicherung einen zuverlässigen Sitz auf, ohne dass ein selbständiges Verdrehen oder Verstellen des Regulierventils auftritt. Zur Realisierung eines entsprechenden Schließverhaltens des Türschließers wird in der Regel ein Dämpfungsfluid verwendet, das entlang von Strömungskanälen geleitet wird. Durch ein entsprechendes Einstellen am Regulierventil kann zumindest an einer Stelle der Strömungsquerschnitt für das Fluid verändert werden, wodurch somit das Schließverhalten, insbesondere die Schließgeschwindigkeit des Türschließers reguliert werden kann. Trotz der wirkenden Kräfte, ausgehend vom strömenden Fluid auf das Regulierventil, sorgt die Verdrehsicherung dafür, dass sich das Regulierventil am Gewinde nicht ungewollt und selbständig verreguliert. Im eingeschraubten Zustand des Regulierventils befindet sich das elastische Element innerhalb der Nut und liegt unmittelbar am Innengewinde der Bohrung des Türschließers an, wodurch eine klemmende Wirkung erzielt wird. Das bedeutet, dass der Spielraum zwischen dem Innengewinde der Bohrung und dem Gewinde des Regulierventils durch das elastische Element vollständig ausgefüllt ist. Hierdurch wird eine hohe Flächenpressung, insbesondere zwischen den Gewindeflanken des Innengewindes und des Regulierventils erzeugt. Es hat sich gezeigt, dass sich durch eine derartige Anordnung die ineinander greifenden Gewindeteile nicht mehr selbständig verdrehen. Ferner ist ein manuelles und/oder gewolltes Verstellen des Regulierventils selbstverständlich weiterhin möglich. In anderen Worten ausgedrückt kann das Regulierventil jederzeit entsprechend den gewünschten Anforderungen bezogen auf ein definiertes Schließverhalten reguliert werden, wobei die Verdrehsicherung ein selbständiges Verdrehen, insbesondere durch auftretende dynamische Belastungen wirkungsvoll verhindert.

In einer möglichen Ausführungsform der Erfindung ist das elastische Element form- und/oder kraft- und/oder stoffflüssig in der Nut angeordnet. Vorteilhafterweise ist das elastische Element in der Nut eingepresst, ohne dass die Gefahr besteht, während des Montagevorganges und/oder des Einstellvorganges des Regulierventils aus der Nut sich zu lösen. Diese am Gewinde angeordnete Verdrehsicherung stellt eine kostengünstige Möglichkeit dar, dem selbständigen Lockern und Lösen des Regulierventils am Gewinde zuverlässig entgegen zu wirken. Hierbei kann das Gewinde ein Säge-, Trapez-, Rund- oder Flachgewinde sein. Die Nut verläuft vorzugsweise parallel zur Drehachse des Regulierventils. Ferner besteht die Möglichkeit, dass die Nut entlang des gesamten Gewindes des Regulierventils sich erstreckt. In einer weiteren Alternative kann das Regulierventil mit mehreren Nuten am Gewinde ausgeführt sein, die beispielsweise 180° oder 120° umfangsseitig versetzt zueinander angeordnet sind. Es hat sich jedoch gezeigt, dass insbesondere bei hydraulischen Türschließern lediglich eine Nut und ein elastisches Element als zuverlässig wirkende Verdrehsicherung ausreichen.

In einer alternativen Ausführungsform der Erfindung weist der Regulierbereich ein freies Ende auf, das dem Grundkörper abgewandt ist und kegelförmig ausgebildet ist. Zudem kann der Türschließer einen Zulaufkanal, einen Ablaufkanal sowie einen kegelförmigen Ventilsitz aufweisen, wobei das Dämpfungsfluid durch den Zulaufkanal entlang des Ventilsitzes zum Ablaufkanal leitbar ist. Das freie Ende des Regulierbereiches ist im Wesentlichen dem Ventilsitz des Türschließers angepasst. In der Regel besteht ein gewisser Abstand zwischen dem Ventilsitz und dem kegelförmigen Ende des Regulierbereiches, so dass das Fluid vom Zulaufkanal zwischen dem Ventilsitz und dem kegelförmigen Ende des Regulierbereiches in Richtung des Ablaufkanals strömen kann.

In einer möglichen Alternative des Regulierventils sind der Grundkörper und der Regulierbereich einstückig und materialeinheitlich ausgebildet.

Ferner ist es selbstverständlich denkbar, den Grundkörper und den Regulierbereich aus zwei unterschiedlichen Materialien herzustellen. In der letztgenannten Ausführungsalternative ist der Regulierbereich am Grundkörper, insbesondere durch ein Spritzgussverfahren angeordnet. Der Regulierbereich besteht aus einem Kunststoffmaterial. Um insbesondere eine hohe Festigkeit zu erzielen, kann der besagte Kunststoff faserverstärkt sein, insbesondere kann der Kunststoff einen bestimmten Anteil an Glasfasern und/oder Kohlenstofffasern und/oder Aramidfasern aufweisen. Der Grundkörper ist aus einem Kunststoff hergestellt. Es hat sich gezeigt, dass durch die Ausbildung des Regulierbereiches mit einem Kunststoff das Schließverhalten des Türschließers - auch bei sich verändernden Temperaturen - im Wesentlichen unverändert bleibt. Ein aus Kunststoff bestehender Grundkörper kann zum Beispiel in einer weiteren Alternative leicht mit einer Farbe versehen werden, so dass ein optischer Unterschied verschiedener Regulierventilarten einfach visualisierbar ist. Vorteilhafterweise sind der Grundkörper und der Regulierbereich durch ein 2K-Spritzgussverfahren miteinander verbunden. In einer möglichen Alternative kann es sinnvoll sein, das elastische Element als Spritzgussteil aus Kunststoff in die Nut einzubringen.

Um einen zuverlässigen Halt des Regulierbereiches am Grundkörper zu gewährleisten, ist der Grundkörper an der dem Ventilsitz zugewandten Seite mit einem Hohlraum ausgebildet, in den sich der Regulierbereich erstreckt. Hierbei kann der Grundkörper mit zumindest einer Öffnung am Hohlraum ausgeführt sein, die durch den Regulierbereich ausgefüllt ist. Der Regulierbereich erstreckt sich somit auch in die Öffnung des Grundkörpers, wodurch die Befestigung des Regulierbereiches am Grundkörper zusätzlich verstärkt wird.

Eine bevorzugte Ausführungsform des Regulierventils sieht vor, dass das Gewinde, das am Grundkörper oder auch am Regulierbereich ausgebildet sein kann, einen Nenndurchmesser D und einen Kerndurchmesser d aufweist, wobei die Nut mit einer Nuttiefe ausgeführt ist, die insbesondere zwischen dem Kerndurchmesser d und dem Nenndurchmesser D liegt. Im montierten Zustand des Regulierventils füllt das elastische Element den Zwischenraum zwischen dem Innengewinde des Türschließers und dem Gewinde des Regulierungsventils aus, wodurch eine erhöhte Flächenpressung an den ineinander greifenden Gewindeteilen erreicht werden kann.

Bei dem elastischen Element kann es sich um einen Kunststofffaden handeln, der beispielsweise in die Nut eingepresst ist. Zweckmässigerweise ist die Nut in das Gewinde durch ein Fräsverfahren bis kurz vor dem Kerndurchmesser eingebracht. In einer Alternative der Erfindung ist der Kunststofffaden als Nylonfaden ausgebildet.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung im Einzelnen beschrieben sind. Dabei können die in den Patentansprüchen und in der Beschreibung erwähnten Merkmale jeweils einzeln für sich oder in beliebiger Kombination erfindungswesentlich sein.

Es zeigen:
- Fig. 1:: Ein Regulierventil mit einer Verdrehsicherung, die eine Nut sowie ein elastisches Element aufweist,
- Fig. 2:: das Regulierventil aus Fig. 1 entlang der Schnittlinie II-II,
- Fig. 3:: eine vergrößerte Darstellung des elastischen Elementes innerhalb der Nut und
- Fig. 4:: das Regulierventil im eingebauten Zustand innerhalb eines hydraulischen Türschließers.

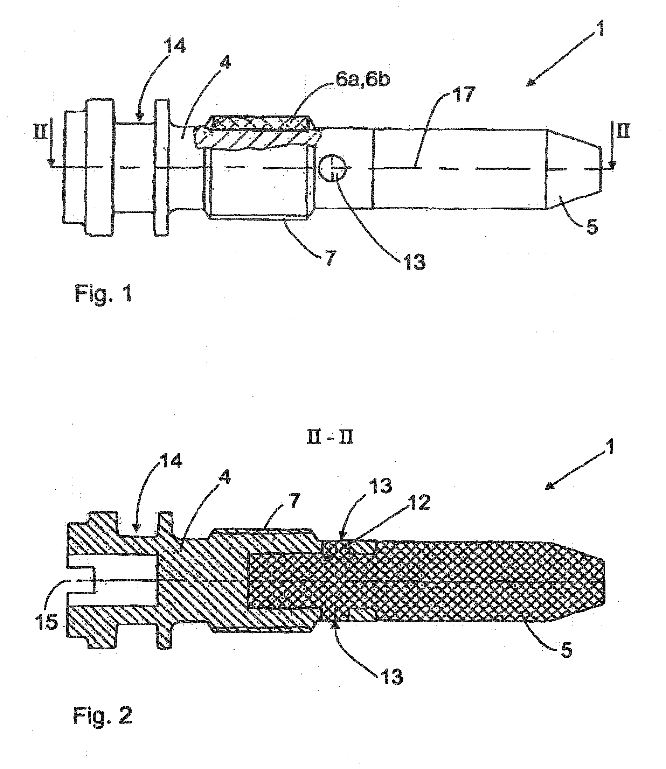

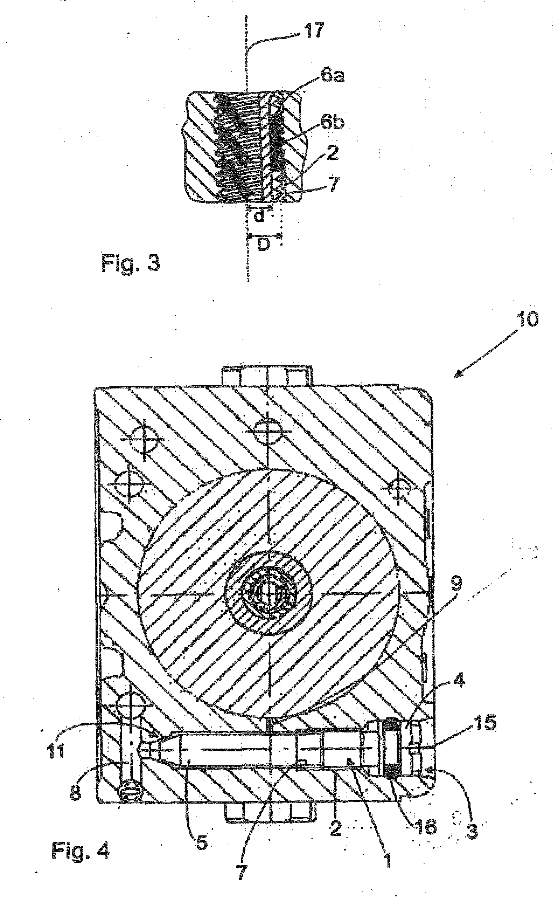

Fig. 1 zeigt ein Regulierventil 1, welches für einen hydraulischen Türschließer vorgesehen ist. Hierbei kann es sich um diverse Türschließer handeln, die beispielsweise mit einer Kurvenexzenterscheibe gemäß DE 103 61 085 A1 oder mit einer Zahnstange gemäß DE 199 01 234 C1 betreibbar sind. Das Regulierventil 1 ist mit einem Grundkörper 4 ausgebildet, der im dargestellten Ausführungsbeispiel aus Metall besteht. Ein metallischer Grundkörper 4 ist von dem Schutzumfang des Patents nicht umfasst. Erfindungsgemäß besteht der Grundkörper aus einem Kunststoff. Am Kopfbereich des Grundkörpers 4 ist ein Schlitz 15 ausgebildet, der in Fig. 2 dargestellt ist. Mit Hilfe dieses Schlitzes 15 kann der Monteur das Regulierventil 1 in eine Bohrung 3 des Türschließers 10, der in Fig. 4 dargestellt ist, eindrehen sowie das Schließverhalten des Türschließers 10 entsprechend regulieren. Des Weiteren ist der Grundkörper 4 mit einem Gewinde 7 ausgestattet, welches mit einem Innengewinde 2 des Türschließers 10 zusammenwirkt. Das Gewinde 7 ist als Feingewinde ausgebildet, das eine Verdrehsicherung aufweist, die aus einer Nut 6a und einem elastischen Element 6b besteht. Die Nut 6a erstreckt sich gemäß Fig. 1 entlang des gesamten Gewindes 7 parallel zur Drehachse 17 des Regulierventils 1. Selbstverständlich kann die Nut 6a sich nur in einem Teilbereich des Gewindes 7 erstrecken, welches in vergrößerter Darstellung gemäß Fig. 3 dargestellt ist.

Zudem weist das Regulierventil 1 einen Regulierbereich 5 auf, der durch ein Spritzgussverfahren am Grundkörper 4 angeordnet ist. Der Regulierbereich 5 besteht aus einem Kunststoffmaterial, welches im vorliegenden Ausführungsbeispiel ein Polyacetal (POM) ist, das sich unter anderem durch seine gute elektrische Eigenschaft, gute Chemikalienfestigkeit und eine hohe Abriebfestigkeit auszeichnet. Der Regulierbereich 5 ist an seinem freien Ende, das dem Grundkörper 4 abgewandt ist, kegelförmig, sich verjüngend ausgeführt.

Der Grundkörper 4 weist an der dem Schlitz 15 abgewandten Seite einen Hohlraum 12 auf, in den der Regulierbereich 5 vollständig sich erstreckt. Zudem sind am Hohlraum 12 zwei kreisförmige Öffnungen 13 angeordnet, die ebenfalls durch den Regulierbereich 5 ausgefüllt sind. Zwischen dem Gewinde 7 und dem Schlitz 15 ist eine Aufnahme 14 für ein Dichtelement 16 vorgesehen, das in Fig. 4 explizit gezeigt ist.

In Fig. 3 weist das Gewinde 7 einen Nenndurchmesser D und einen Kerndurchmesser d auf, wobei die Nut 6a, die im Gewinde 7 eingefräst ist, eine Nuttiefe hat, die zwischen dem Kerndurchmesser d und dem Nenndurchmesser D liegt. Beim Einschrauben des Regulierventils 1 in die Bohrung 3 wird eine zusätzliche klemmende Wirkung durch die erfindungsgemäße Verdrehsicherung 6a, 6b erzielt. Wie besonders Fig. 3 verdeutlicht, wird der axiale Spielraum zwischen dem Gewinde 7 des Regulierventils 1 und dem Innengewinde 2 der Bohrung 3 durch das elastische Element 6b ausgefüllt. Hierdurch wird eine hohe Flächenpressung, insbesondere an den verdrehsicherungsfreien Gewindeflanken der ineinander greifenden Gewindeteile 2, 7 erreicht, welches durch die Pfeildarstellungen in Fig. 3 angedeutet ist. Das in der Nut 6a eingepresste elastische Element 6b ist ein Kunststofffaden 6b. Der besondere Vorteil dieser beschriebenen Verdrehsicherung 6a, 6b ist, dass diese eine selbständige Verregulierung des Regulierventils 1 zuverlässig verhindert. Gleichzeitig kann das Regulierventil 1 manuell jederzeit verdreht und/oder aus der Bohrung 3 ausgeschraubt werden.

Gemäß Fig. 4 befindet sich das freie Ende des Regulierbereiches 5 in einem kegelförmigen Ventilsitz 11 des Türschließers 10. Im Bereich des kegelförmigen Endes des Regulierbereiches 5 befindet sich ein Zulaufkanal 8, durch den ein Öl geführt wird. In der Regel liegt das kegelförmige Ende des Regulierbereiches 5 nicht unmittelbar am Ventilsitz 11 auf, so dass das Öl durch den zwischen dem Ventilsitz 11 und dem freien kegelförmigen Ende des Regulierbereiches 5 sich bildenden schmalen Kanal in Richtung eines Ablaufkanals 9 strömen kann. Am Regulierventil 1 kann der Strömungsquerschnitt für das Öl eingestellt werden, wodurch insbesondere das Schließverhalten des Türschließers 10 beeinflussbar ist. Die durch das strömende Öl auf das Regulierventil 1 wirkenden Kräfte können aufgrund der beschriebenen Verdrehsicherung 6a, 6b auch im Toleranzbereich keine ungewollte Verdrehung des Regulierventils 1 auslösen.

## Patentansprüche

1. Regulierventil (1), das insbesondere in eine mit einem Innengewinde (2) ausgeführte Bohrung (3) eines hydraulischen Türschließers (10) befestigbar ist, mit einem Grundkörper (4), der einen Regulierbereich (5) aufweist, einer Verdrehsicherung und einem Gewinde (7), das in das Innengewinde (2) der Bohrung (3) anordbar ist,
wobei
die Verdrehsicherung zumindest eine Nut (6a) aufweist, die am Gewinde (7) angeordnet ist und ein elastisches Element (6b) aufnimmt, wobei der Grundkörper (4) an der dem Ventilsitz (11) zugewandten Seite einen Hohlraum (12) aufweist, in den sich der Regulierbereich (5) erstreckt und der Regulierbereich (5) am Grundkörper (4) befestigt ist,
wobei der Grundkörper (4) und der Regulierbereich (5) aus einem Kunststoffmaterial bestehen, insbesondere der Grundkörper (4) aus einem faserverstärkten Kunststoff besteht.

2. Regulierventil (1) nach Patentanspruch 1, **dadurch gekennzeichnet, dass** das elastische Element (6b) form- und/oder kraft- und/oder stoffschlüssig in der Nut (6a) angeordnet ist.

3. Regulierventil (1) nach Patentanspruch 1 oder 2, **dadurch gekennzeichnet, dass** der Regulierbereich (5) ein freies Ende aufweist, das dem Grundkörper (4) abgewandt ist und kegelförmig ausgebildet ist.

4. Regulierventil (1) nach einem der vorhergehenden Patentansprüche, **dadurch gekennzeichnet, dass** der Regulierbereich (5) am Grundkörper (4) durch ein Spritzgussverfahren befestigt ist.

5. Regulierventil (1) nach einem der vorhergehenden Patentansprüche, **dadurch gekennzeichnet, dass** der Grundkörper (4) farblich ausgeführt ist.

6. Regulierventil (1) nach einem der vorhergehenden Patentansprüche, **dadurch gekennzeichnet, dass** das Gewinde (7) einen Nenndurchmesser D und einen Kerndurchmesser d aufweist, wobei die Nut (6a) mit einer Nuttiefe ausgeführt ist, die insbesondere zwischen dem Kerndurchmesser d und dem Nenndurchmesser D liegt.

7. Regulierventil (1) nach einem der vorhergehenden Patentansprüche, **dadurch gekennzeichnet, dass** das elastische Element (6b) ein Kunststofffaden (6b) ist.

8. Regulierventil (1) nach einem der vorhergehenden Patentansprüche, **dadurch gekennzeichnet, dass** der Türschließer (10) einen Zulaufkanal (8), einen Ablaufkanal (9) und einen kegelförmigen Ventilsitz (11) aufweist, wobei ein Fluid durch den Zulaufkanal (8) entlang des Ventilsitzes (11) zum Ablaufkanal (9) leitbar ist.

9. Regulierventil (1) nach Patentanspruch 9, **dadurch gekennzeichnet, dass** der Grundkörper (4) mit zumindest einer Öffnung (13) am Hohlraum (12) ausgeführt ist, die durch den Regulierbereich (5) ausgefüllt ist.

10. Regulierventil (1) nach einem der vorhergehenden Patentansprüche, **dadurch gekennzeichnet, dass** das elastische Element (6b) ein Spritzgussteil aus Kunststoff ist.

## Claims

1. A regulating valve (1), which in particular is attachable in a bore (3) of a hydraulic door closer (10) executed with a female thread (2), which valve has a body (4) having a regulating area (5), has an anti-rotation protection, and a thread (7), which can be disposed in the female thread (2) of the bore (3),
wherein
the anti-rotation protection has at least one groove (63), which is disposed at the thread (7) and receives an elastic element (6b), the body (4), at the side oriented towards the valve seat (11), having a hollow space (12), into which the regulating area (5) extends, and wherein the regulating area (5) is attached to the body (4),
wherein the body (4) and the regulating area (5) consist of plastic material, and in particular in that the body (4) consists of a fibre reinforced plastic material.

2. A regulating valve (1) according to patent claim 1, **characterized in that** the elastic element (6b) is disposed positively and/or non-positively and/or positively by material in the groove (6a).

3. A regulating valve (1) according to patent claim 1 or 2, **characterized in that** the regulating area (5) has a free end, which is facing away from the body (4) and is formed in a conical shape.

4. A regulating valve (1) according to one of the preceding patent claims, **characterized in that** the regulating area (5) is attached to the body (4) by an injection moulding process.

5. A regulating valve (1) according to one of the preceding patent claims, **characterized in that** the body (4) is executed in colours.

6. A regulating valve (1) according to one of the preceding patent claims, **characterized in that** the thread (7) has a nominal diameter D and a core diameter d, the groove (6a) being executed with a groove depth value, which is in particular between the core diameter d and the nominal diameter D.

7. A regulating valve (1) according to one of the preceding patent claims, **characterized in that** the elastic element (6b) is a plastic material strand (6b).

8. A regulating valve (1) according to one of the preceding claims, **characterized in that** the door closer (10) presents a supply channel (8), a drainage channel (9) and a cone-shaped valve seat (11), wherein a fluid can be conducted through the supply channel (8) along the valve seat (11) to the drainage channel (9).

9. A regulating valve (1) according to patent claim 9, **characterized in that** the body (4) is executed with at least one opening (13) at the hollow space (12), which opening is filled by the regulating area (5).

10. A regulating valve (1) according to one of the preceding patent claims, **characterized in that** the elastic element (6b) is an injection moulding part made from plastic material.

## Revendications

1. Vanne de réglage (1), qui peut être fixée en particulier dans une forure (3) d'un ferme-porte hydraulique (10) qui est aménagée avec un taraudage (2), avec un corps (4) présentant une plage de réglage (5), avec une protection anti-rotation et avec un filetage (7) qui peut être agencé dans le taraudage (2) de la forure (3),
dans laquelle
la protection anti-rotation présente au moins une rainure (6a) qui est agencée au filetage (7) et reçoit un élément élastique (6b), le corps (4), sur le côté orienté vers le siège de vanne (11), présentant un espace creux (12) dans lequel s'étend la plage de réglage (5), et la plage de réglage (5) est fixée au corps (4),
dans laquelle le corps (4) et la plage de réglage (5) sont faits en matière plastique, en particulier le corps (4) est fait de matière plastique renforcée par des fibres.

2. Vanne de réglage (1) selon la revendication 1, **caractérisée en ce que** l'élément élastique (6b) est disposé dans la rainure (6a) par la forme et/ou par la force et/ou par la matière.

3. Vanne de réglage (1) selon la revendication 1 ou 2, **caractérisée en ce que** la plage de réglage (5) présente une extrémité libre qui est détournée du corps (4) et aménagée en forme de cône.

4. Vanne de réglage (1) selon l'une des revendications précédentes, **caractérisée en ce que** la plage de réglage (5) est fixée au corps (4) par un procédé de moulage par injection.

5. Vanne de réglage (1) selon l'une des revendications précédentes, **caractérisée en ce que** le corps (4) est aménagé en couleur.

6. Vanne de réglage (1) selon l'une des revendications précédentes, **caractérisée en ce que** le filetage (7) possède un diamètre nominal D et un diamètre de noyau d, la rainure (6a) étant aménagée avec une profondeur de rainure qui se situe en particulier entre le diamètre de noyau d et le diamètre nominal D.

7. Vanne de réglage (1) selon l'une des revendications précédentes, **caractérisée en ce que** l'élément élastique (6b) est un fil en matière plastique (6b).

8. Vanne de réglage (1) selon l'une des revendications précédentes, **caractérisée en ce que** le ferme-porte (10) présente un canal d'arrivée (8), un canal de décharge (9) et un siège de vanne (11) en forme de cône, un fluide pouvant être guidé à travers le canal d'arrivée (8) long du siège de vanne (11) vers le canal de décharge (9).

9. Vanne de réglage (1) selon la revendication 9, **caractérisée en ce que** le corps (4) est aménagé avec au moins une ouverture (13) à l'espace creux (12), laquelle est remplie par la plage de réglage (5).

10. Vanne de réglage (1) selon l'une des revendications précédentes, **caractérisée en ce que** l'élément élastique (6b) est une pièce injectée en matière plastique.
